# EUROPEAN PATENT APPLICATION

(11) **EP 2 626 021 A1**
(43) Date of publication of application: **14.08.2013**
(21) Application number: 13000478.1
(22) Date of filing: 31.01.2013
(51) Int. Cl.: A61B 18/14

(54) **Electrode tip for radiofrequency tissue ablation and electrode having the same**

(30) Priority: 10.02.2012 KR 20120013701
(71) Applicant: Jun, Myong-Ki, Gwacheon-Shi Gyeonggi-Do 427-804 (KR)
(72) Inventor: Jun, Myong-Ki, Gwacheon-Shi Gyeonggi-Do 427-804 (KR)
(74) Representative: Müller - Hoffmann & Partner

(57) **Abstract**

ELECTRODE TIP FOR RADIOFREQUENCY TISSUE ABLATION AND ELECTRODE HAVING THE SAME

The present invention relates to an electrode tip for radiofrequency tissue ablation and an electrode, wherein a hollow portion and an outlet, through which the saline solution introduced into the hollow portion is introduced into the living body, are formed in the electrode tip to cool the electrode tip with the saline solution, while allowing the saline solution to flow into the tissue to increase electrical conductivity to prevent carbonization of the tissue, which makes it possible to cauterize the tissue in a relatively larger area within a shorter time or with a higher output.

## Description

### TECHNICAL FIELD

The present invention relates to an electrode tip for radiofrequency tissue ablation and an electrode having the same, and more particularly, to an electrode tip for radiofrequency tissue ablation and an electrode having the same, wherein a hollow portion is formed in the electrode tip to cool the electrode tip with the saline solution, while increasing electrical conductivity to prevent carbonization of the tissue and cauterizing the tissue in a larger area.

### BACKGROUND ART

In general, a technique of inserting an elongated hollow-tube type electrode into the living tissue in a piercing fashion and coagulating or ablating the target living tissue with radiofrequency energy has been already well-known. In this case, when a radiofrequency output is applied to the living tissue, the living tissue is heated such that the living tissue and the blood vessel are coagulated via rather complex biochemical mechanism, this process being mainly performed by coagulation of cells (including tissue, blood vessel, and blood) caused by thermal deformation of protein in the cells at a temperature of at least about 55 °C.

However, the electrode for radiofrequency tissue ablation has disadvantages in that the blood adjacent to the electrode is coagulated enough to be carbonized, that the carbonized living tissue adjacent to the electrode acts as an insulator, which interrupts expansion of the living tissue coagulation area, and that in the case of some organs such as the thyroid gland, the tissue is burned to settle down on the electrode, which causes serious pain to patients. In order to solve the foregoing problems, while the saline solution for cooling is circulated, some of it is discharged to the outside to be able to coagulate the living tissue in a wide area.

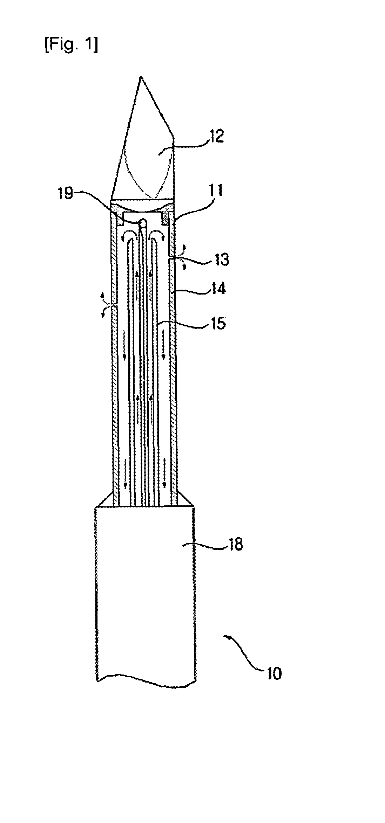

FIG. 1 is a view showing an example of a conventional electrode for radiofrequency tissue ablation.

An electrode portion 10 of the electrode for radiofrequency tissue ablation includes an electrode 11, an electrode tip 12, an outlet 13, an outlet tube 14, an inlet tube 15, and a temperature sensor 19. The electrode for radiofrequency tissue ablation is formed in a mono-polar type or a bi-polar type, and the conventional electrode for radiofrequency tissue ablation shown in FIG. 1 is formed in a mono-polar type. The electrode 11 is hollow and has a pointed end so that the electrode 11 can penetrate through the skin into the living body with the closed electrode tip 12. The inlet tube 15, through which the saline solution is introduced, is provided inside the electrode 11, and the outlet tube 14, which has a larger diameter than the inlet tube 15 is provided outside the electrode 11. The saline solution introduced through the inlet tube 15 turns back around the end of the electrode and flows out of the electrode portion 10 through the outlet tube 14. Here, at least one outlet 13 is formed in the outlet tube 14 so that some of the saline solution introduced through the inlet tube 15 is injected into the living body. As the saline solution is injected into the living body to prevent carbonization of the tissue, it is possible to cauterize the living tissue in a relatively larger area for a longer time, or with a higher output even for the same time. Meanwhile, a temperature sensor 19 may be further included in the electrode 11 to measure the temperature in the electrode 11.

However, the conventional electrode for radiofrequency tissue ablation has disadvantages in that, since the electrode tip 12 of the electrode for radiofrequency tissue ablation is not provided with a passage for cooling, or since a hole through which the saline solution is injected into the living body is not provided even if the passage for cooling the electrode tip 12 is provided, energy is concentrated on the electrode tip 12 during the radiofrequency tissue ablation to thereby easily carbonize the heated tissue.

### DISCLOSURE OF THE INVENTION

An object of the present invention is to provide an electrode tip for radiofrequency tissue ablation and an electrode having the same, wherein the electrode tip is provided with an outlet for the saline solution so that the saline solution can be introduced therethrough.

Another object of the present invention is to provide an electrode tip for radiofrequency tissue ablation and an electrode having the same, which can control the amount of the saline solution to be introduced through the electrode tip for radiofrequency tissue ablation.

According to an aspect of the present invention, there is provided an electrode tip for radiofrequency tissue ablation, comprising: a body having a pointed front end and a stepped rear end, the pointed front end being formed by a plurality of slant surfaces, the stepped rear end being stepped enough to be inserted into a pipe; a hollow portion disposed from the rear end of the body to a certain depth so that the saline solution can be introduced therethrough; and an outlet for discharging the saline solution which is the coolant introduced into the hollow portion.

In one embodiment of the present invention, the hollow portion is disposed from the rear end of the body toward the front end with a constant diameter and extended to a position where the hollow portion has a larger diameter than the body to form a hole, so that the saline solution can be discharged to the outside of the body through the hole.

In another embodiment of the present invention, the electrode tip for radiofrequency tissue ablation further includes a through hole disposed from the hollow portion to the slant surface of the front end to discharge the saline solution to the outside of the body.

In a further embodiment of the present invention, the electrode tip for radiofrequency tissue ablation further includes a filter disposed in the hollow portion.

According to another aspect of the present invention, there is provided an electrode for radiofrequency tissue ablation, comprising: an electrode composed of a hollow tube; a coolant passage provided in the electrode to cool the electrode with the coolant flowing therethrough; and an electrode tip including a body having a pointed front end and a stepped rear end, the pointed front end being formed in a slanted fashion, the stepped rear end being stepped enough to be inserted into the electrode, and a hollow portion disposed from the rear end of the body to a certain depth so that the coolant can be introduced therethrough.

In one embodiment of the present invention, the electrode tip further includes an outlet for discharging the saline solution which is the coolant introduced into the hollow portion.

In another embodiment of the present invention, the electrode for radiofrequency tissue ablation further includes a pipe inserted into the hollow portion of the electrode tip to supply the saline solution, and a syringe pump for supplying the saline solution through the pipe.

In a further embodiment of the present invention, the electrode includes a fine hole for discharging the saline solution which is the coolant.

In a still further embodiment of the present invention, the electrode tip includes an outlet for discharging the saline solution which is the coolant introduced into the hollow portion.

In a still further embodiment of the present invention, the electrode tip includes a filter disposed in the hollow portion to reduce the water pressure of the saline solution.

The electrode tip for radiofrequency tissue ablation and the electrode having the same according to the present invention can cool the electrode tip with the saline solution and introduce the saline solution into the living body through the electrode tip, so that it is possible to cool the electrode tip with the saline solution, while allowing the saline solution to flow into the tissue to increase electrical conductivity to prevent carbonization of the tissue, thereby cauterizing the tissue in a larger area for a shorter time or with a higher output.

In addition, the electrode tip for radiofrequency tissue ablation and the electrode having the same according to the present invention can control the amount of the saline solution injected through the electrode tip, by using the syringe pump.

### BRIEF DESCRIPTION OF THE DRAWINGS

FIG. 1 is a view showing a conventional electrode for radiofrequency tissue ablation.
FIGS. 2 to 4 are views showing an electrode tip for radiofrequency tissue ablation according to a first embodiment of the present invention.
FIGS. 5 to 7 are views showing an electrode tip for radiofrequency tissue ablation according to a second embodiment of the present invention.
FIG. 8 is a view showing an electrode for radiofrequency tissue ablation according to a first embodiment of the present invention.
FIG. 9 is a view showing an electrode for radiofrequency tissue ablation according to a second embodiment of the present invention.
FIG. 10 is a view showing an electrode for radiofrequency tissue ablation according to a third embodiment of the present invention.
FIG. 11 is a view showing an electrode for radiofrequency tissue ablation according to a fourth embodiment of the present invention.
FIG. 12 is a view showing an electrode for radiofrequency tissue ablation according to a fifth embodiment of the present invention.

### BEST MODE FOR CARRYING OUT THE INVENTION

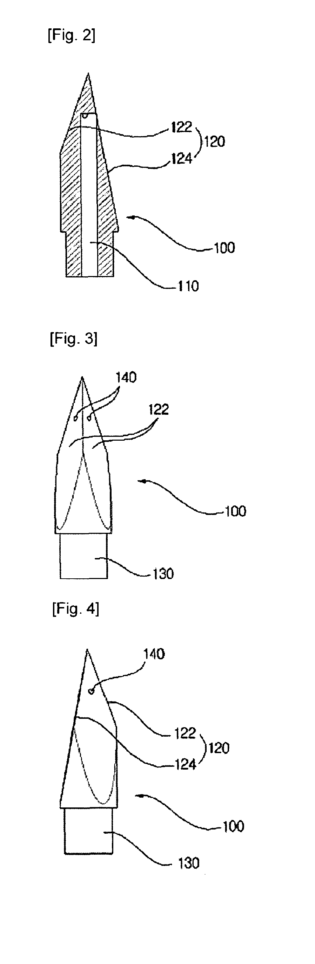

FIGS. 2 to 4 are views showing an electrode tip for radiofrequency tissue ablation according to a first embodiment of the present invention.

A body of the electrode tip 100 for radiofrequency tissue ablation according to the first embodiment of the present invention has a front pointed end formed by slant surfaces 122 and 124; 120, so that the electrode can be easily inserted into the living tissue, and has a rear stepped end 130 so as to be inserted into a hollow body such as a pipe. A hollow portion 110 is formed in the body, so that the coolant can be introduced therethrough into the electrode tip 100 for radiofrequency tissue ablation to cool the same. When a hole 140 is formed in the slant surface 122 of the electrode tip 100 for radiofrequency tissue ablation to allow the coolant to flow from the hollow portion 110 to the living body to be cauterized, the saline solution should be used as the coolant. The hollow portion 110 is disposed from the rear end of the body toward the front end with a constant diameter. If the hollow portion 110 reaches a certain depth, it pierces through the slant surface 120 to form the hole 140 therein.

The slant surfaces 120 include slant surfaces 122, which define a more acute angle relative to the central axis of the body, and slant surfaces 124, which define a more obtuse angle relative to the central axis of the body. The slant surfaces 122 defining a more acute angle are adjacent to each other, while the slant surfaces 124 defining a more obtuse angle are adjacent to each other. When the hollow portion 110 reaches a certain depth, two holes 140 are formed in the slant surfaces 122 defining a more acute angle. When the hollow portion 110 exceeds a certain depth, holes 140 are formed both in the slant surfaces 122 defining a more acute angle and the slant surfaces 124 defining a more obtuse angle, as a result of which a total of four holes 140 can be formed. It would be appreciated that the number of the slant surfaces 120 and the angle of the slant surface 120 relative to the central axis of the body can vary.

To manufacture the electrode tip 100 for radiofrequency tissue ablation, a rear stepped end 130 is formed at a cylindrical material, and a hollow portion 110 is formed from the rear end to a certain depth by means of discharge machining or super drilling. Then, slant surfaces 120 are machined to form a front pointed end, and at the same time, the end of the hollow portion 110 is exposed through the slant surfaces 120 to form holes 140.

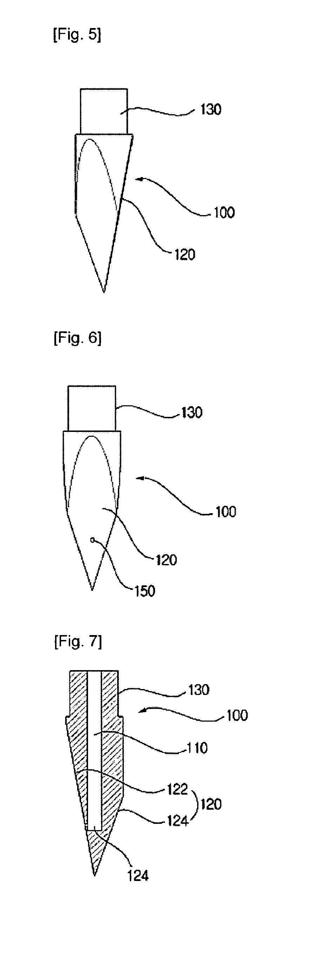

FIGS. 5 to 7 are views showing an electrode tip for radiofrequency tissue ablation according to a second embodiment of the present invention.

A body of the electrode tip 100 for radiofrequency tissue ablation according to the second embodiment of the present invention also has a front pointed end, which is formed by slant surfaces 122 and 124; 120, and a rear stepped end 130, which is stepped to be inserted into a hollow body such as a pipe. In addition, as in the first embodiment, a hollow portion 110 is formed that introduces the coolant into the body.

Unlike the first embodiment, the hollow portion 110 of the electrode tip 100 for radiofrequency tissue ablation according to the second embodiment does not reach a certain depth to pierce through the slant surface 120. Instead, a through hole 150 is disposed from the outside of the slant surface 120 to the hollow portion 110. This through hole 150 makes it possible to flow the saline solution (coolant) into the living body and efficiently cauterize the living tissue using radiofrequency, while cooling the electrode tip 100 for radiofrequency tissue ablation on which the radiofrequency energy is concentrated. As a result, this prevents carbonization of the tissue to be cauterized.

To manufacture the electrode tip 100 for radiofrequency tissue ablation, a rear stepped end 130 is formed at a cylindrical material, and a hollow portion 110 is formed from the rear end to a certain depth by means of discharge machining or super drilling. Then, slant surfaces 120 are machined to form a front pointed end, and a through hole 150 is formed from the slant surface 120 to the hollow portion 110 by means of a laser. The slant surfaces 120 can be formed after the formation of the through hole 150.

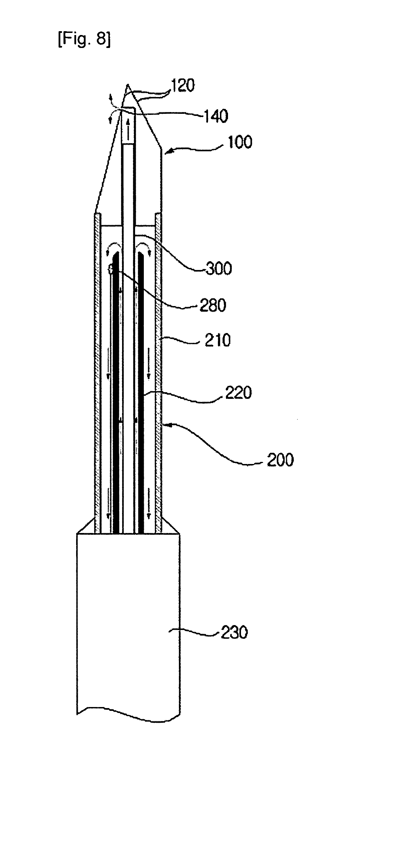

FIG. 8 is a view showing an electrode for radiofrequency tissue ablation according to a first embodiment of the present invention.

The electrode for radiofrequency tissue ablation according to the first embodiment of the present invention includes an electrode tip 100 and an electrode 200. The electrode tip 100 is identical to the electrode tip for radiofrequency tissue ablation according to the first embodiment of the present invention shown in FIGS. 2 to 4. The electrode 200 is formed in a so-called cool type with an outer pipe 210 and an inner pipe 220, the coolant being introduced through the inner pipe 220 and discharged to the outside between the inner pipe 220 and the outer pipe 210 to cool the electrode 200. When the coolant is discharged again between the outer pipe 210 and the inner pipe 220, it must have a high water pressure. If the coolant of high water pressure is discharged to the living body through a hole 140, it may hurt the living body. Accordingly, the electrode may include a pipe 300 for supplying the saline solution to the hollow portion 110 in the electrode tip 100 at a moderate pressure, and a syringe pump (not shown) for supplying the saline solution through the pipe 300. In addition, the electrode includes a temperature sensor 280 for measuring the temperature. It would be appreciated that, instead of the pipe 300 and the syringe pump (not shown), the electrode may include a filter (not shown) or a fin (not shown) which can reduce the water pressure in the hollow portion 110 to discharge the saline solution at a moderate pressure. When the electrode includes the pipe 300 and the syringe pump (not shown), it is possible to control the amount of the saline solution injected into the living body through the electrode tip 100. As such, if the tissue to be cauterized, such as a tumor, is not cauterized well, the amount of the saline solution to be injected can be increased to facilitate the cauterization, and if the amount of cauterization needs to be controlled precisely, the amount of the saline solution to be injected can be decreased.

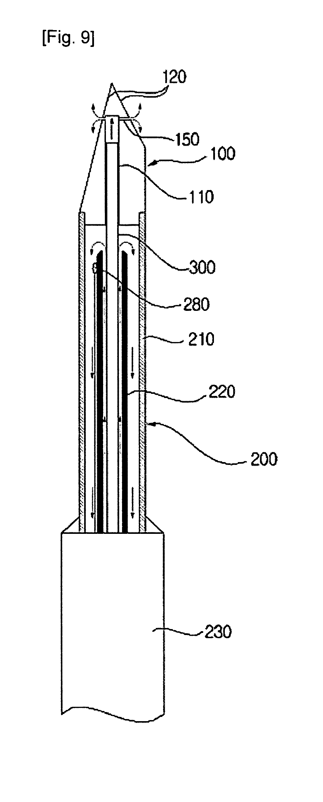

FIG. 9 is a view showing an electrode for radiofrequency tissue ablation according to a second embodiment of the present invention.

The electrode for radiofrequency tissue ablation according to the second embodiment of the present invention includes an electrode tip 100 and an electrode 200. The electrode tip 100 is identical to the electrode tip for radiofrequency tissue ablation according to the second embodiment of the present invention shown in FIGS. 5 to 7. The electrode 200 is formed in a cool type like the electrode 200 of the first embodiment.

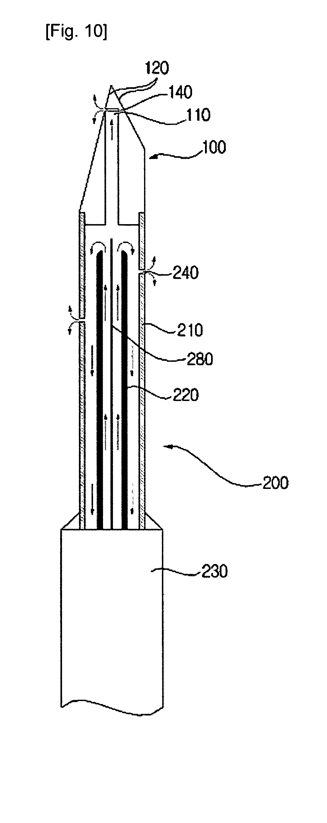

FIG. 10 is a view showing an electrode for radiofrequency tissue ablation according to a third embodiment of the present invention.

The electrode for radiofrequency tissue ablation according to the third embodiment of the present invention includes an electrode tip 100 and an electrode 200. The electrode tip 100 is identical to the electrode tip for radiofrequency tissue ablation according to the second embodiment of the present invention shown in FIGS. 2 to 4. The electrode 200 is formed in a cooled-wet type with an outer pipe 210 and an inner pipe 220, some of the coolant introduced into the inner pipe 220 being discharged through a fine hole 240 formed in the outer pipe 210, some of which being circulated and discharged through the passage between the inner pipe 220 and the outer pipe 210. Although not illustrated in FIG. 10, the electrode may include a pipe 300 and a syringe pump (not shown), as shown in FIGS. 8 and 9, to cool the electrode tip 100 through the hollow portion 110 of the electrode tip 100 and supply the saline solution at a moderate pressure that will be discharged to the living body through the hole 140, and may include a filter (not shown) disposed in the hollow portion 110 to reduce the water pressure of the saline solution. The electrode further includes a temperature sensor 280 for measuring the temperature.

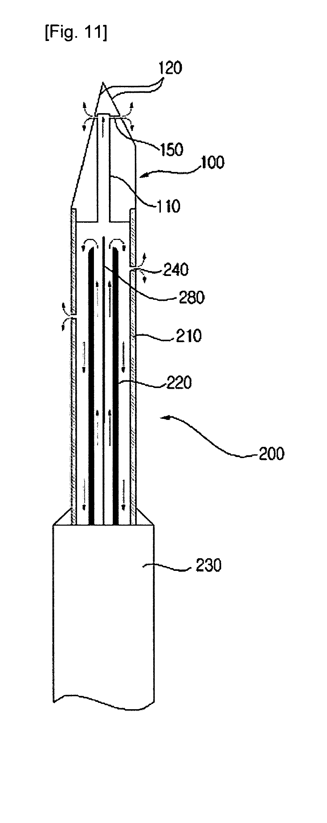

FIG. 11 is a view showing an electrode for radiofrequency tissue ablation according to a fourth embodiment of the present invention.

The electrode for radiofrequency tissue ablation according to the fourth embodiment of the present invention includes an electrode tip 100 and an electrode 200. The electrode tip 100 is identical to the electrode tip for radiofrequency tissue ablation according to the second embodiment of the present invention shown in FIGS. 5 to 7. The electrode 200 is formed in a cooled-wet type like the electrode 200 for radiofrequency tissue ablation according to the third embodiment.

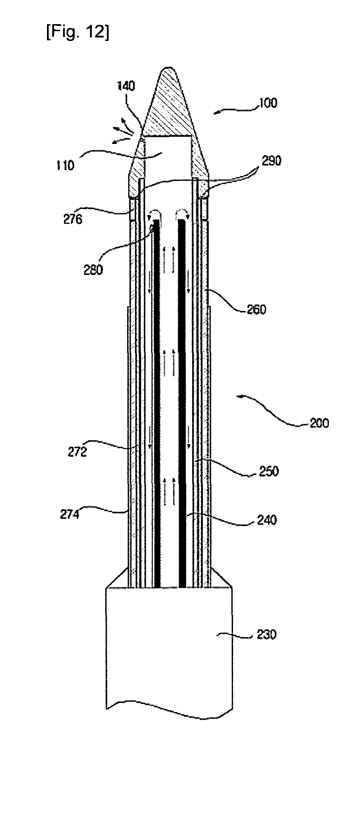

FIG. 12 is a view showing an electrode for radiofrequency tissue ablation according to a fifth embodiment of the present invention.

The electrode for radiofrequency tissue ablation according to the fifth embodiment of the present invention includes an electrode tip 100 and an electrode 200. The electrode tip 100 is identical to the electrode tip for radiofrequency tissue ablation according to the first embodiment of the present invention shown in FIGS. 2 to 4, and the electrode 200 is formed in a bi-polar type. The bi-polar type electrode 200 includes an inner pipe 240, an intermediate pipe 250, and an outer pipe 260, the intermediate pipe 250 and the electrode tip 100 being welded to each other by means of welding. The electrode tip 210 has an opposite polarity to the outer pipe 260, for which reason it is called a bi-polar type. In order to electrically insulate the electrode tip 100 from the outer pipe 260, an inner insulating film 272 is attached between the intermediate pipe 250 and the outer pipe 260, an outer insulating film 274 is attached to the outer rear portion of the outer pipe 260, and an insulating member 276 is attached between the electrode tip 100 and the outer pipe 260. In the meantime, a temperature sensor 280 is attached to the outside of the inner pipe 240, but may also be attached to the inside of the inner pipe 240.

FIG. 12 illustrates that the bi-polar type electrode 200 is combined with the electrode tip 100 for radiofrequency tissue ablation according to the first embodiment of the present invention shown in FIGS. 2 to 4, but the bi-polar type electrode 200 may be combined with the electrode tip 100 for radiofrequency tissue ablation according to the second embodiment of the present invention shown in FIGS. 5 to 7.

## Claims

1. An electrode tip for radiofrequency tissue ablation, comprising:
a body having a pointed front end and a stepped rear end, the pointed front end being formed by a plurality of slant surfaces, the stepped rear end being stepped enough to be inserted into a pipe;
a hollow portion disposed from the rear end of the body to a certain depth so that the saline solution can be introduced therethrough; and
an outlet for discharging the saline solution which is the coolant introduced into the hollow portion.

2. The electrode tip according to claim 1, wherein the hollow portion is disposed from the rear end of the body toward the front end with a constant diameter, and the outlet is formed on the slant surface as the hollow portion pierces through the slanted pointed end.

3. The electrode tip according to claim 1 or 2, wherein the outlet further comprises a through hole disposed from the hollow portion to the slant surface of the front end.

4. The electrode tip according to any one of claims 1 to 3, wherein the electrode tip further comprises a filter disposed in the hollow portion.

5. An electrode for radiofrequency tissue ablation, comprising:
an electrode composed of a hollow tube;
a coolant passage provided in the electrode to cool the electrode with the coolant flowing therethrough; and
an electrode tip including a body having a pointed front end and a stepped rear end, the pointed front end being formed in a slanted fashion, the stepped rear end being stepped enough to be inserted into the electrode, a hollow portion disposed from the rear end of the body to a certain depth so that the coolant can be introduced therethrough, and an outlet for discharging the saline solution introduced into the hollow portion.

6. The electrode according to claim 5, wherein the electrode further comprises:
a pipe inserted into the hollow portion of the electrode tip to supply the saline solution; and
a syringe pump for supplying the saline solution through the pipe.

7. The electrode according to claim 5 or 6, wherein the electrode comprises a fine hole for discharging the saline solution which is the coolant.

8. The electrode according to claim 7, wherein the electrode tip comprises an outlet for discharging the saline solution which is the coolant introduced into the hollow portion.

9. The electrode according to claim 8, wherein the electrode tip comprises a filter disposed in the hollow portion to reduce the water pressure of the saline solution.
